(19) Europäisches Patentamt
European Patent Office
Office européen des brevets

(11) Veröffentlichungsnummer: **0 173 958**
**A2**

(12) # EUROPÄISCHE PATENTANMELDUNG

(21) Anmeldenummer: **85110835.7**

(22) Anmeldetag: **19.08.85**

(51) Int. Cl.⁴: **C 07 D 239/42**
C 07 D 239/46, A 01 N 47/36
//C07D239/48, C07D239/52,
C07D417/12, C07D213/75,
C07D251/20, C07D251/22,
C07D251/46, C07D251/52

(30) Priorität: **30.08.84 DE 3431927**
**08.05.85 DE 3516435**

(43) Veröffentlichungstag der Anmeldung:
**12.03.86 Patentblatt 86/11**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Diehr, Hans-Joachim, Dr.**
**Höhe 35**
**D-5600 Wuppertal 1(DE)**

(72) Erfinder: **Fest, Christa, Dr.**
**Im Johannistal 20**
**D-5600 Wuppertal 1(DE)**

(72) Erfinder: **Kirsten, Rolf, Dr.**
**Carl-Langhans-Strasse 27**
**D-4019 Monheim(DE)**

(72) Erfinder: **Kluth, Joachim, Dr.**
**Kurt-Schumacher-Strasse 9**
**D-4018 Langenfeld(DE)**

(72) Erfinder: **Müller, Klaus-Helmut, Dr.**
**Bockhackstrasse 55**
**D-4000 Düsseldorf 13(DE)**

(72) Erfinder: **Pfister, Theodor, Dr.**
**Lichtenberger Strasse 30**
**D-4019 Monheim(DE)**

(72) Erfinder: **Priesnitz, Uwe, Dr.**
**Severinstrasse 58**
**D-5650 Solingen 1(DE)**

(72) Erfinder: **Riebel, Hans-Jochem, Dr.**
**In der Beek 92**
**D-5600 Wuppertal 1(DE)**

(72) Erfinder: **Roy, Wolfgang, Dr.**
**Walter-Kolb-Strasse 47**
**D-4018 Langenfeld(DE)**

(72) Erfinder: **Santel, Hans-Joachim, Dr.**
**Gerstenkamp 19**
**D-5000 Koeln 80(DE)**

(72) Erfinder: **Schmidt, Robert R., Dr.**
**Im Waldwinkel 110**
**D-5060 Bergisch-Gladbach 2(DE)**

(54) **1-(2-Oxyaminocarbonylphenylsulfonyl)-3-heteroaryl-harnstoffe.**

(57) Die Erfindung betrifft neue 1-(2-Oxyamino-carbonylphenylsulfonyl)-3-heteroaryl-harnstoffe der allgemeinen Formel

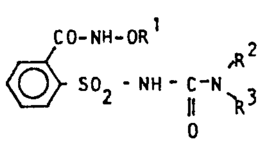

(I)

in welcher

$R^1$ für einen gegebenenfalls substituierten Rest aus der Reihe Alkyl, Alkenyl, Alkinyl, Cycloalkyl, Cycloalkylalkyl, Aralkyl un Aryl steht,

$R^2$ für Wasserstoff oder für einen gegebenenfalls substituierten Rest aus der Reihe Alkyl, Alkenyl, Alkinyl und Aralkyl steht und

./...

EP 0 173 958 A2

$R^3$ für einen gegebenenfalls substituierten und/oder gegebenenfalls anellierten sechsgliedrigen aromatischen Heterocyclus, welcher wenigstens ein Stickstoffatom enthält, steht,

ein Verfahren zu ihrer Herstellung und ihre Verwendung als Herbizide.

BAYER AKTIENGESELLSCHAFT
Konzernverwaltung RP
Patentabteilung

5090 Leverkusen, Bayerwerk

Bi/Kü-c
IVb

1-(2-Oxyaminocarbonylphenylsulfonyl)-3-heteroaryl-harnstoffe

Die Erfindung betrifft neue 1-(2-Oxyaminocarbonylphenylsulfonyl)-3-heteroaryl-harnstoffe, ein erfinderisches Verfahren zu deren Herstellung und deren Verwendung als Herbizide.

Es ist bekannt, daß bestimmte 1-Arylsulfonyl-3-heteroarylharnstoffe, wie z.B. 1-(2-Methoxy-phenylsulfonyl)-3-(4,6-dimethyl-pyrimidin-2-yl)-harnstoff, herbizid wirksam sind. Die Wirkung dieser Verbindungen ist jedoch nicht immer ganz befriedigend (vergl. US-PS 4 169 719).

Es wurden nun neue 1-(2-Oxyaminocarbonylphenylsulfonyl)-3-heteroaryl-harnstoffe der allgemeinen Formel (I)

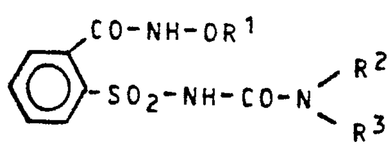

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für einen gegebenenfalls substituierten und/oder gegebenenfalls anellierten sechsgliedrigen aromatischen Heterocyclus, welcher wenigstens ein Stickstoffatom enthält, steht,

gefunden.

Man erhält die neuen Verbindungen der Formel (I) nach einem erfinderischen Verfahren, wenn man Heterocyclen der Formel (II)

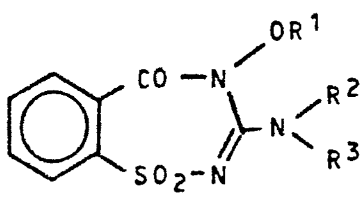

(II)

in welcher

$R^1$, $R^2$ und $R^3$ die oben angegebenen Bedeutungen haben.

mit Wasser in Gegenwart von Mineralsäuren und gegebenenfalls in Gegenwart von Verdünnungsmitteln bei Temperaturen zwischen 0°C und 100°C umsetzt.

Die neuen 1-(2-Oxyaminocarbonylphenylsulfonyl)-3-heteroaryl-harnstoffe der Formel (I) zeichnen sich durch starke herbizide Wirksamkeit aus.

Überraschenderweise zeigen die neuen Verbindungen der Formel (I) erheblich bessere herbizide Wirkung als viele bekannte chemische Verbindungen gleicher Wirkungsrichtung.

Es ist weiter als überraschend anzusehen, daß die erfindungsgemäßen Verbindungen der Formel (I) durch selektive Ringöffnung von Heterocyclen der Formel (II) hergestellt werden können, da neben dieser neuartigen Reaktion auch andere Ringöffnungen, beispielsweise durch Angriff an der Carbonyl- oder an der Sulfonylgruppierung zu erwarten waren.

Gegenstand der Erfindung sind vorzugsweise Verbindungen der Formel (I), in welcher

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl, oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht, in welcher weiter

$R^2$ für Wasserstoff, $C_1$-$C_4$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] steht, in welcher ferner

$R^3$ für den Rest 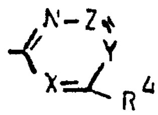 steht, worin

$R^4$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht,

X für Stickstoff oder eine Methin-brücke (CH) steht,

Y für Stickstoff oder eine gegebenenfalls substituierte Methin-brücke C-$R^5$ steht, wobei

$R^5$ für Wasserstoff, Fluor, Chlor, Brom, Cyano, Formyl, $C_1$-$C_4$-Alkyl [welches gegebenenfalls durch Fluor und/oder Chlor substituiert ist], für $C_1$-$C_4$-Alkylcarbonyl oder $C_1$-$C_4$-Alkoxy-carbonyl steht, und

Z für Stickstoff oder eine gegebenenfalls substituierte Methin-brücke C-$R^6$ steht, wobei

$R^6$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht.

Gegenstand der Erfindung sind insbesondere Verbindungen der Formel (I) in welcher

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff steht und

$R^3$ für den Rest 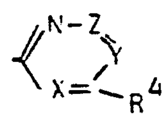 steht, worin

$R^4$ für Chlor, Methyl, Ethyl, Methoxy, Difluormethoxy oder Ethoxy steht,

X für Stickstoff steht,

Y für eine Methin-brücke (CH) steht, und

Z für eine gegebenenfalls substituierte Methin-brücke C-$R^6$ steht, wobei

$R^6$ für Wasserstoff, Chlor, Methyl, Methoxy, Ethoxy, Methylthio, Ethylthio, Dimethylamino oder Diethylamino steht.

Die beim erfindungsgemäßen Verfahren ablaufende chemische Reaktion kann beispielsweise durch folgendes Formelschema skizziert werden:

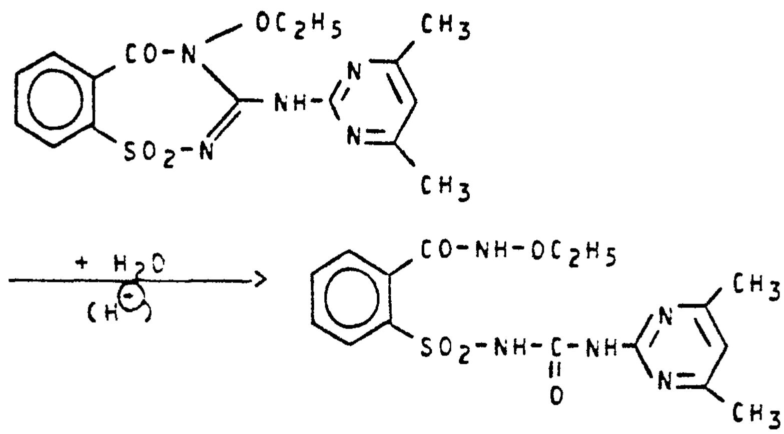


Die beim erfindungsgemäßen Verfahren als Ausgangsverbindungen zu verwendenden Heterocyclen sind durch die Formel (II) allgemein definiert.

In Formel (II) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für Ausgangsstoffe der Formel (II) sind in der nachstehenden Tabelle 1 aufgeführt.

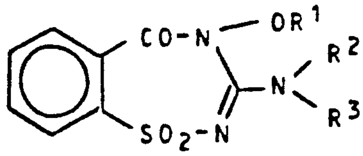

(II)

<u>Tabelle 1:</u> Beispiele für Ausgangsstoffe der Formel (II)

| $R^1$ | $R^2$ | $R^3$ |
|---|---|---|
| $CH_3$ | H | 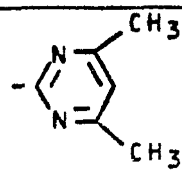 |
| $CH_3$ | $CH_3$ | 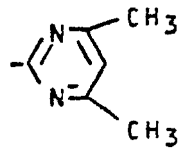 |
| $C_2H_5$ | H | 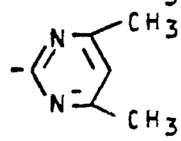 |
| $C_2H_5$ | $CH_3$ | 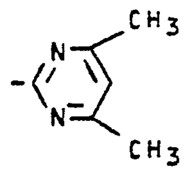 |
| $C_3H_7$-n | H | 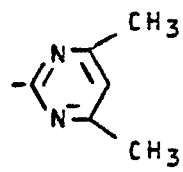 |
| $C_3H_7$-n | $CH_3$ | 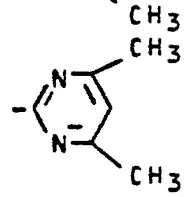 |
| $C_3H_7$-iso | H | 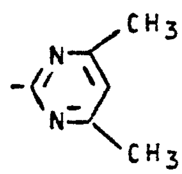 |
| $C_3H_7$-iso | $CH_3$ | 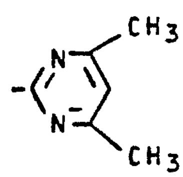 |

Tabelle 1(Fortsetzung)

| $R^1$ | $R^2$ | $R^3$ |
|---|---|---|
| $C_4H_9$-n | H | 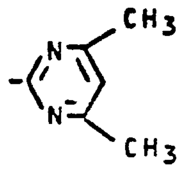 |
| $C_4H_9$-n | $CH_3$ | 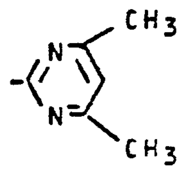 |
| $C_4H_9$-sec. | H | 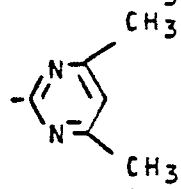 |
| $C_4H_9$-sec. | $CH_3$ | 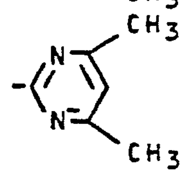 |
| $C_4H_9$-iso | H | 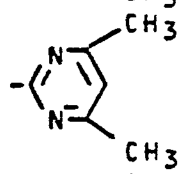 |
| $C_4H_9$-iso | $CH_3$ | 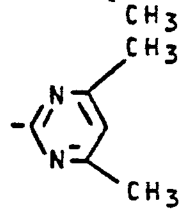 |
| $C_8H_{17}$-n | H | 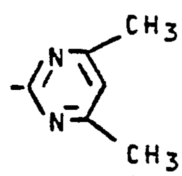 |
| $C_8H_{17}$-n | $CH_3$ | 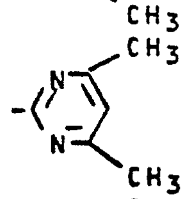 |
| $-CH_2$ 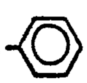 | H | 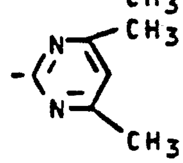 |

Tabelle 1: (Fortsetzung)

| $R^1$ | $R^2$ | $R^3$ |
|---|---|---|
| 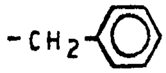 | $CH_3$ | 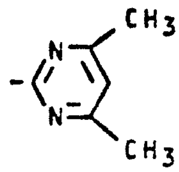 |
| 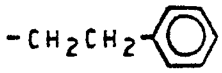 | H | 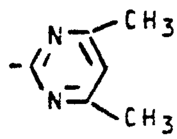 |
| 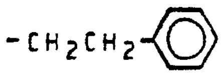 | $CH_3$ | 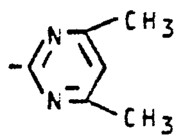 |
| 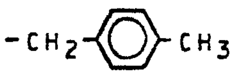 | H | 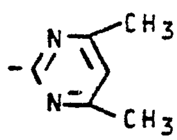 |
| 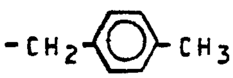 | $CH_3$ | 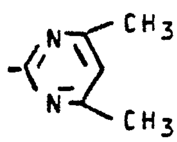 |
| 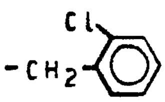 | H | 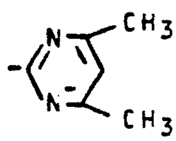 |
| 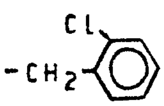 | $CH_3$ | 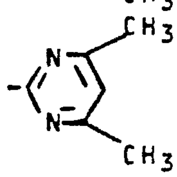 |
| 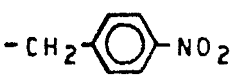 | H | 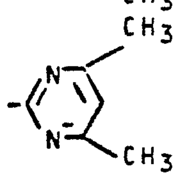 |

0173958

Die Verbindungen der Formel (II) sind bisher nicht in der Literatur beschrieben. Man erhält die Verbindungen der Formel (II), wenn man 2-Chlorsulfonylbenzoylchlorid der Formel (III)

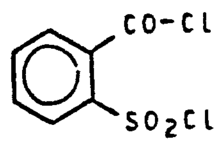

(III)

mit Oxyguanidin-Derivaten der Formel (IV)

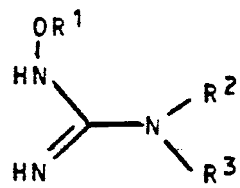

(IV)

in welcher

$R^1$, $R^2$ und $R^3$ die oben angegebene Bedeutung haben,

in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid, Chloroform, Tetrahydrofuran oder Dioxan, bei Temperaturen zwischen -30°C und +50°C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende 2-Chlorsulfonylbenzoylchlorid der Formel (III) ist bereits bekannt (vgl. DE-OS 2 036 171).

Die weiter als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituenten-definition der Formel (I) vorzugsweise bzw. als insbesondere bevorzugt angegeben sind.

Als Ausgangsstoffe der Formel (IV) seien beispielsweise genannt:

N'-(4-Methyl-pyrimidin-2-yl)-, N'-(4-Ethyl-pyrimidin-2-yl)-, N'-(4,6-Dimethoxy-pyrimidin-2-yl)-, N'-(4,6-Diethoxy-pyrimidin-2-yl)-, N'-(4-Difluormethoxy-6-methyl-pyrimidin-2-yl)-, N'-(4,6-Dimethyl-pyrimidin-2-yl)-, N'-4-Methoxy-6-methyl-pyrimidin-2-yl)-, N'-(4-Ethoxy-6-methyl-pyrimidin-2-yl)-, N'-(4-Chor-6-methoxy-pyrimidin-2-yl)-, N'-(4-Chlor-6-ethoxy-pyrimidin-2-yl)-, N'-(4-Chlor-6-dimethylamino-pyrimidin-2-yl)-, N'-(4-Methyl-6-methylthio-pyrimidin-2-yl)- und N'-(4-Dimethylamino-6-methyl-pyrimidin-2-yl)-N''-methoxy-guanidin, -N''-ethoxy-guanidin, -N''-propoxy-guanidin, -N''-isopropoxyguanidin, -N''-butoxy-guanidin, -N''-isobutoxy-guanidin, -N''-sec.-butoxy-guanidin, -N''-pentoxy-guanidin, -N''-isopentoxy-guanidin, -N''-hexyloxy-guanidin, -N''-octyloxy-guanidin, -N''-allyloxy-guanidin, -N''-(2-chlor-ethoxy)-guanidin, -N''(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy)-guanidin, -N''-methoxycarbonylmethoxy-guanidin, -N''-ethoxycarbonyl-methoxy-guanidin, -N''-(1-methoxycarbonyl-ethoxy)-guanidin, -N''-(1-ethoxycarbonylethoxy)-guanidin, -N''-Dimethylaminocarbonylmethoxy-guanidin, -N''-(2-phenyl-ethoxy)-guanidin, -N''-phenoxy-guanidin, -N''-(4-methyl-benzyl-oxy)-guanidin, -N''-(4-fluor-benzyloxy)-guanidin, -N''-(4-chlor-benzyloxy)-guanidin, -N''-(4-nitrobenzyloxy)-guanidin, -N''-(2,6-dichlor-benzyloxy)-guanidin, -N''-(4-methoxycarbonyl-benzyloxy)-guanidin und -N''-(4-ethoxycarbonyl-benzyloxy)-guanidin.

Die Ausgangsstoffe der Formel (IV) sind zum Teil bekannt (vgl. J.Chem. Soc. 1962, 3915 und DE-OS 3 334 455).

Man erhält die Verbindungen der Formel (IV) wenn man Cyanamid-Derivate der Formel (V)

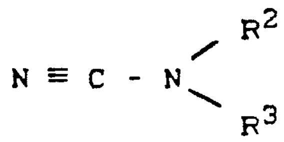

(V)

in welcher

$R^2$ und $R^3$ die oben angegebenen Bedeutungen haben.

mit Hydroxylamin-Derivaten der Formel (VI)

$$H_2N\text{-}OR^1 \qquad (VI)$$

in welcher

$R^1$ die oben angegebenen Bedeutungen hat,

bzw. mit Hydrochloriden von Hydroxylamin-Derivaten der Formel (VI)

gegebenenfalls in Gegenwart von Verdünnungsmitteln, wie z.B. Ethanol, Propanol oder Butanol, bei Temperaturen zwischen 20°C und 120°C umsetzt und gegebenenfalls die Umsetzungsprodukte mit Säureakzeptoren, wie z.B. Ammoniak. Kaliumcarbonat oder Natriumhydroxid, behandelt.

Die Cyanamid-Derivate der Formel (V) sind zum Teil bekannt (vergl. J. Chem. Soc. 1953, 1725). Man erhält die Verbindungen der Formel (V) im wesentlichen nach folgenden Synthesewegen:

(a) durch Umsetzung von Alkalimetall- oder Erdalkalimetall-Salzen von Cyanamid - wie z.B. Natriumcyanamid oder Calciumcyanamid - mit Chlor-hetarenen der Formel (VII)

$$Cl\text{-}R^3 \qquad (VII)$$

in welcher

$R^3$ die oben angegebene Bedeutung hat,

und gegebenenfalls anschließend - wenn $R^2$ nicht für Wasserstoff steht - mit Halogenverbindungen der Formel (VIII)

$$Q\text{-}R^2 \qquad (VIII)$$

in welcher

$R^2$ für einen gegebenenfalls substituierten Rest aus der Reihe Alkyl, Alkenyl, Alkinyl und Aralkyl steht, und

Q für Chlor, Brom oder Jod steht,

gegebenenfalls in Gegenwart von inerten Verdünnungsmitteln, wie z.B. Aceton, Acetonitril oder Dimethylformamid, bei Temperaturen zwischen 0°C und 100°C.

Nach Einengen und Auflösen des Rückstandes in Wasser können die Cyanamid-Derivate der Formel (V) durch Ansäuren, z.B. mit Salzsäure, ausgefällt und durch Absaugen isoliert werden.

Alternativ erhält man die Verbindungen der Formel (V)

(b) für den Fall, daß $R^3$ für einen substituierten Pyrimidinylrest steht, durch Umsetzung von Cyanoguanidin ('Dicyandiamid') mit ß-Dicarbonylverbindungen oder deren Derivaten, wie Acetylaceton (vergl. J. Chem. Soc. <u>1953</u>, 1725-1730); Acetessigsäureestern (vergl. J. Prakt. Chem. <u>77</u> (1908), 542 und J. Chem. Soc. <u>1948</u>, 586) oder Malonsäureestern (vergl. DE-PS 158 591).

Die aus Acetessigsäureestern bzw. Malonsäureestern erhaltenen 2-Cyanamino-4-hydroxy-6-methyl- bzw. -4,6-dihydroxy-pyrimidine können auf bekannte Weise durch Umsetzung mit Alkylierungsmitteln, wie z.B. Dimethyl- oder Diethylsulfat, gegebenenfalls in Gegenwart von Verdünnungsmitteln, wie z.B. Wasser, Methanol, Ethanol, n- und iso-Propanol, Aceton, Dioxan oder Dimethylformamid, und in Gegenwart von Säurebindemitteln, wie z.B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z.B. Acetanhydrid oder Acetylchlorid acyliert und nach der Alkylierung mit wäßrigen Säuren oder Basen wieder entacyliert.

In einem weiteren Alternativverfahren erhält man die Verbindungen der Formel (V), wenn man

(c) Amino-hetarene der Formel (IX)

$$H_2N\text{-}R^3 \qquad (IX)$$

in welcher

$R^3$ die oben angegebene Bedeutung hat,

mit Carbonylisothiocyanaten der Formel (X)

$$\begin{array}{c} O \\ \| \\ R^7\text{-}C\text{-}N{=}C{=}S \end{array} \qquad (X)$$

in welcher

$R^7$ für Ethoxy oder Phenyl steht,

gegebenenfalls in Gegenwart eines inerten Verdünnungsmittels, wie z.B. Aceton, Acetonitril oder Toluol, bei Temperaturen zwischen 0°C und 100°C umsetzt, die hierbei gebideten Carbonylthioharnstoffe der Formel (XI)

$$\begin{array}{c} \quad O \quad\;\; S \\ \quad \| \quad\;\;\; \| \\ R^7\text{-}C\text{-}NH\text{-}C\text{-}NH\text{-}R^3 \end{array} \qquad (XI)$$

in welcher

$R^3$ und $R^7$ die oben angegebenen Bedeutungen haben,

gegebenenfalls nach Einengen durch Absaugen isoliert und mit wäßrigen Alkalimetall- oder Erdalkalimetall-hydroxidlösungen, wie z.B. Natronlauge, gegebenenfalls in Gegenwart eines organischen Lösungsmittels, wie z.B. Tetrahydrofuran oder Dioxan, bei Temperaturen zwischen 0°C und 120°C umsetzt und die nach Ansäuren, z.B. mit Salzsäure, kristallin erhaltenen Thioharnstoffe der Formel (XII)

$$H_2N-\overset{\overset{S}{\|}}{C}-NH-R^3 \qquad (XII)$$

in welcher

$R^3$ die oben angegebene Bedeutung hat,

durch Absaugen isoliert und mit Metallverbindungen, welche Schwefelwasserstoff binden können, wie z.B. mit Blei(II)-acetat, Kupfer(II)-acetat, Quecksilber-(II)-acetat oder Eisen(II)-acetat, in Gegenwart von wäßrigen Alkalimetall- oder Erdalkalimetall-hydroxidlösungen, wie z.B. Natronlauge, bei Temperaturen zwischen 20°C und 100°C umsetzt, nach Ende der Umsetzung filtriert und das Filtrat mit einer Säure, wie z.B. Essigsäure, ansäuert. Die hierbei kristallin anfallenden Produkte der Formel (V) können durch Absaugen isoliert werden.

Die Ausgangsstoffe für die vorausgehend unter (a), (b) und (c) beschriebenen Herstellungsverfahren für die Cyanamid-Derivate der Formel (V) sind bekannt und/oder können nach an sich bekannten Verfahren hergestellt werden.

Hierzu gehören die Chlor-hetarene der Formel (VII) (vergl. J. Chem. Soc. (c) 1966, 2031: Chem. Pharm. Bull. 11 (1963), 1382-1388 und Arch. Pharm. 295, (1962), 649-657), die Halogenverbindungen der Formel (VIII) (handelsübliche Chemikalien), die Amino-hetarene der Formel (IX) (vgl. Chem. Pharm. Bull. 11, (1963): J. Chem. Soc. 1946, 81 und US-PS 4 299 960) und die Carbonylisothiocyanate der Formel (X) (vgl. J. Heterocycl. Chem. 5, (1968), 837 und US-PS 4 160 037).

Das erfindungsgemäße Verfahren zur Herstellung der neuen Verbindungen der Formel (I) wird vorzugsweise in Wasser als Lösungsmittel durchgeführt. Als weitere Verdünnungsmittel kommen alle inerten organischen Lösungsmittel, vorzugsweise jedoch aprotische polare Solventien in Betracht. Hierzu gehören gegebenenfalls Ketone, wie z.B. Aceton und Methylethylketon, Nitrile, wie z.B. Acetonitril und Propionsäurenitril, Dimethylsulfoxid, Sulfolan und 1,2-Dimethoxyethan.

Das erfindungsgemäße Verfahren wird in Gegenwart von Mineralsäuren durchgeführt. Beispiele hierfür sind Salzsäure, Schwefelsäure und Phosphorsäure. Vorzugsweise wird Salzsäure verwendet.

Die Reaktionstemperaturen können beim erfindungsgemäßen Verfahren innerhalb eines größeren Bereiches variiert werden. Im allgemeinen arbeitet man zwischen 0°C und +100°C, vorzugsweise zwischen 10°C und +50°C. Das erfindungsgemäße Verfahren wird im allgemeinen bei Normaldruck durchgeführt.

Zur Durchführung des erfindungsgemäßen Verfahrens setzt man je Mol des Heterocyclus der Formel (II) im allgemeinen zwischen 1 und 25 Mol, vorzugsweise zwischen 1 und 10 Mol Salzsäure ein.

Die Reaktionskomponenten werden gewöhnlich bei Raumtemperatur zusammengegeben und das Reaktionsgemisch wird bis zum Reaktionsende gerührt.

Die Aufarbeitung und Isolierung der neuen Verbindungen der Formel (I) kann nach üblichen Methoden durchgeführt werden. Beispielsweise wird das Reaktionsgemisch nach dem Reaktionsende eingeengt. Das im Rückstand verbleibende Produkt der Formel (I) wird durch Verreiben mit einem geeigneten organischen Lösungsmittel, wie z.B. Ethanol zur Kristallisation gebracht und durch Absaugen isoliert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die

an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2- Benzthiazolyl)-N,N'-dimethyl-harnstoff, 3-(3-Chlor-4- methylphenyl)-1,1-dimethylharnstoff, 3-(4-isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-

2,4-(1H,3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylester, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,005 und 10 kg pro ha.

Die Herstellung und die Verwendung der erfindungsgemäßen Wirkstoffe geht aus den nachfolgenden Beispielen hervor.

## Herstellungsbeispiele:

### Beispiel 1

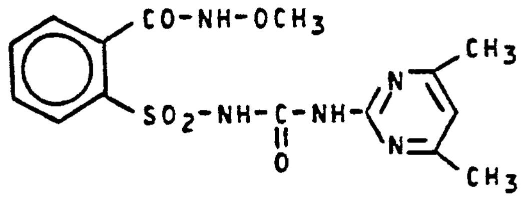

2,5 g (0,007 Mol) des Heterocyclus folgender Strukturformel

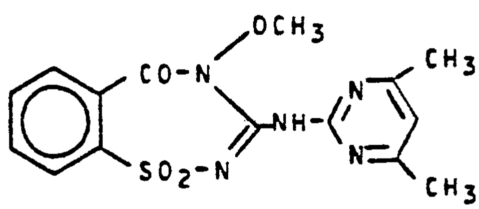

30 ml Wasser und 5 ml konzentrierte Salzsäure werden 15 Stunden bei 20-25°C gerührt. Dann wird dieses Gemisch eingedampft und der Rückstand mit Ethanol verrieben. Das hierbei kristallin ausgefallene Produkt wird durch Absaugen isoliert.

Man erhält 0,8 g (30 % der Theorie) 1-(2-Methoxyaminocarbonylphenylsulfonyl)-3-(4,6-dimethylpyrimidin-2-yl)-harnstoff vom Schmelzpunkt 218°C.

Nach dem im vorausgehenden Beispiel exemplarisch beschriebenen Verfahren können die in der nachstehenden Tabelle 2 aufgeführten Verbindungen der Formel (I) hergestellt werden:

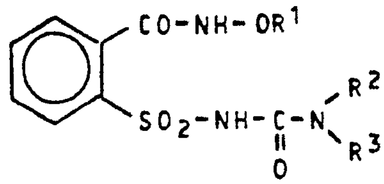

(I)

Tabelle 2

| Bsp. Nr. | $R^1$ | $R^2$ | $R^3$ | Schmelz-punkt(°C) |
|---|---|---|---|---|
| 2 | $-C_8H_{17}-n$ | H | 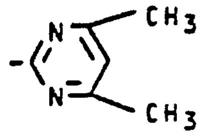 | 165 |
| 3 | $-C_4H_9-n$ | H | 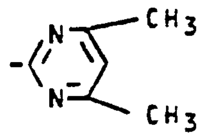 | |
| 4 | $-CH_2-$ 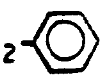 | H | 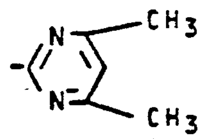 | |
| 5 | $-C_2H_5$ | H | 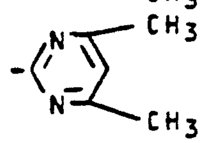 | |
| 6 | $-C_3H_7-n$ | H | 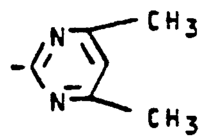 | |
| 7 | $-C_3H_7-i$ | H | 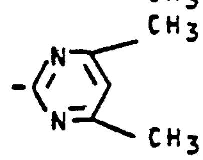 | |
| 8 | $-C_4H_9-i$ | H | 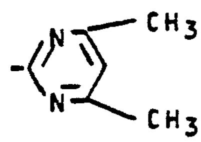 | |
| 9 | $-CH_2CH_2-$ 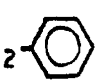 | H | 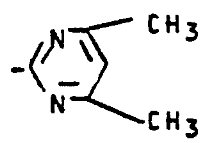 | |
| 10 | $-CH_2CH=CH_2$ | H | 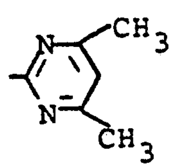 | 177 (Zers.) |

**Tabelle 2** Fortsetzung

| Bsp. Nr. | $R^1$ | $R^2$ | $R^3$ | Schmelzpunkt (°C) |
|---|---|---|---|---|
| 11 | $-CH_3$ | H | 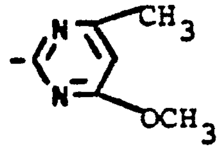 | 173 |
| 12 | $-CH_3$ | H | 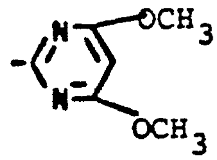 | |
| 13 | $-CH_2CH(CH_3)_2$ | H | 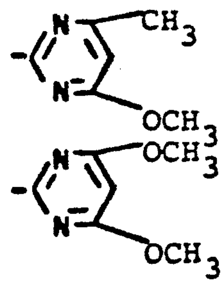 | |
| 14 | $-C_2H_5$ | H | | |
| 15 | $-CH_3$ | H | 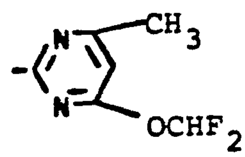 | |
| 16 | $-CH_2-CH=CH_2$ | H | 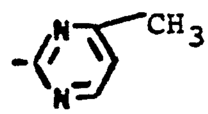 | |
| 17 | $-CH_3$ | H | 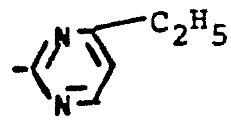 | |
| 18 | $-C_3H_7n$ | H | 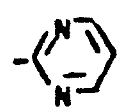 | |
| 19 | $-CH_2-COOC_2H_5$ | H | 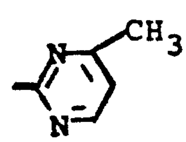 | |

Tabelle 2 Fortsetzung

| Bsp. Nr. | $R^1$ | $R^2$ | $R^3$ | Schmelzpunkt(°C) |
|---|---|---|---|---|
| 20 | $-CH_3$ | H | 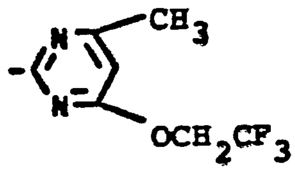 | |
| 21 | $CH_3$ | H | 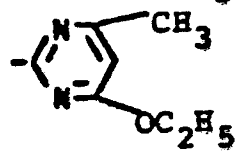 | |
| 22 | $CH_3$ | H | 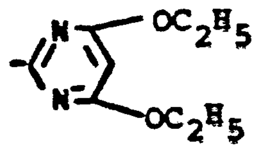 | |
| 23 | $C_2H_5$ | H | 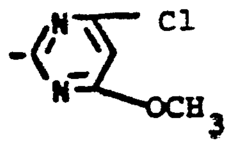 | |
| 24 | $CH_3$ | H | 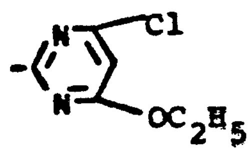 | |
| 25 | $CH_3$ | H | 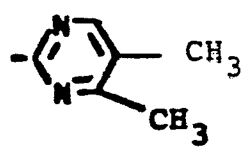 | |

Herstellung von Ausgangsprodukten der Formel (II):

Beispiel II-1

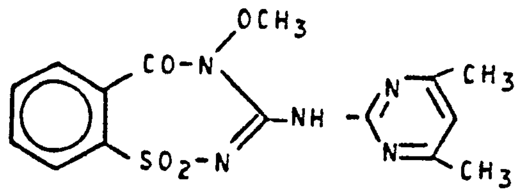

Eine Lösung von 60 g (0,25 Mol) 2-Chlorsulfonylbenzoylchlorid in 50 ml Methylenchlorid wird unter Rühren bei -10°C bis -5°C zu einer Mischung von 49 g (0,25 Mol) N'-(4,6-Dimethylpyrimidin-2-yl)-N''-methoxyguanidin, 50 g (0,63 Mol) Pyridin und 200 ml Methylenchlorid getropft. Man rührt dann 3 Stunden bei 20 bis 25°C nach. Nach dem Waschen der Methylenchloridlösung mit verdünnter Salzsäure, Eiswasser, Trocknen, Filtrieren und Einengen erhält man einen Rückstand, der durch Verreiben mit Ethanol zur Kristallisation gebracht wird. Das hierbei kristallin angefallene Produkt wird durch Absaugen isoliert.

Man erhält 65 g (72 % der Theorie) der Verbindung der oben angegebenen Strukturformel vom Schmelzpunkt 185°C.

Nach dem im vorausgehenden Beispiel II-1 exemplarisch beschriebenen Verfahren können die in der nachstehenden Tabelle 3 aufgeführten Verbindungen der Formel (II) hergestellt werden:

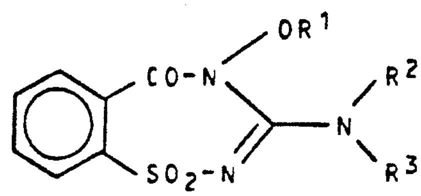

(II)

Tabelle 3

| Bsp. Nr. | $R^1$ | $R^2$ | $R^3$ | Schmelzpunkt(°C) |
|---|---|---|---|---|
| (II-2) | $-CH_2$- 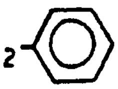 | H | 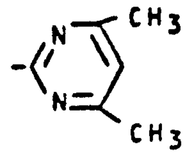 | 162 |
| (II-3) | $-C_8H_{17}$ | H | 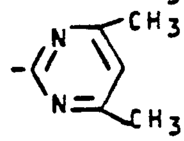 | 136 |
| (II-4) | $-C_4H_9$ | H | 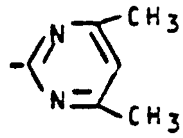 | amorph |
| (II-5) | $-C_2H_5$ | H | 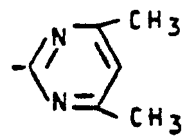 | 175 |
| (II-6) | $-C_3H_7$-n | H | 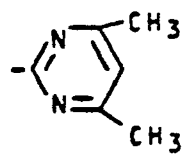 | 81 |
| (II-7) | $-C_3H_7$-i | H | 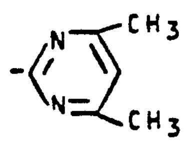 | 155 |
| (II-8) | $-C_4H_9$-i | H | 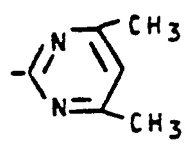 | |
| (II-9) | $-CH_2CH_2$- 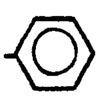 | H | 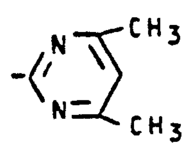 | |
| (II-10) | $-CH_2-CH=CH_2$ | H | 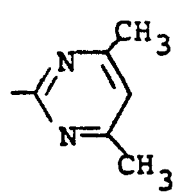 | 153-156 |

**Tabelle 3** Fortsetzung

| Bsp. Nr. | $R^1$ | $R^2$ | $R^3$ | Schmelzpunkt(°C) |
|---|---|---|---|---|
| (II-11) | $-CH_3$ | H | 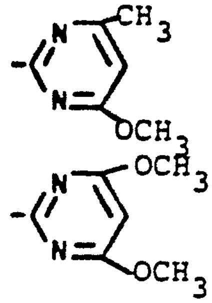 | 166 |
| (II-12) | $-CH_3$ | H | | 180 |
| (II-13) | $-CHCH_2CH_3$<br>$CH_3$ | H | 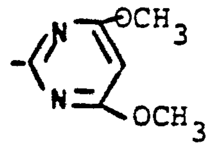 | |
| (II-14) | $-CH_3$ | H | 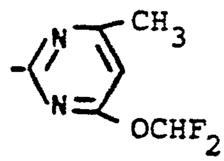 | |
| (II-15) | $-CH_3$ | H | 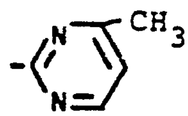 | 173 |
| (II-16) | $-CH_2$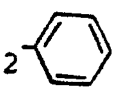 | H | 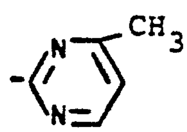 | |
| (II-17) | $-CH_3$ | H | 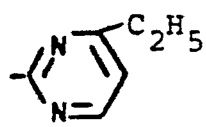 | |
| (II-18) | $-CH_3$ | H | 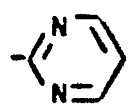 | 110 |

Tabelle 3 Fortsetzung

| Bsp. Nr. | $R^1$ | $R^2$ | $R^3$ | Schmelzpunkt(°C) |
|---|---|---|---|---|
| (II-19) | $-C_2H_5$ | H | 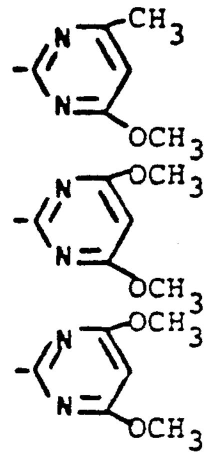 | |
| (II-20) | $-C_2H_5$ | H | | |
| (II-21) | $-CH_2-$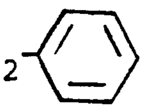 | H | | |
| (II-22) | $-CH_2COOC_2H_5$ | H | 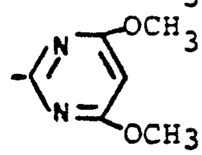 | |
| (II-23) | $-CH_3$ | H | 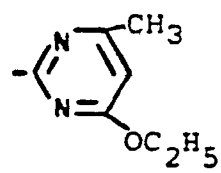 | |
| (II-24) | $-CH_3$ | H | 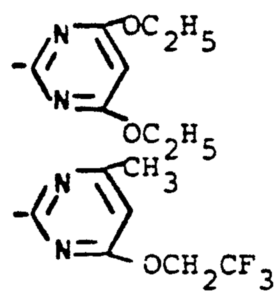 | |
| (II-25) | $-CH_3$ | H | | |
| (II-26) | $-CH_2-CH=CH_2$ | H | 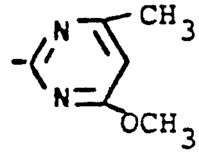 | |

Herstellung der Ausgangsstoffe der Formel (IV)

Beispiel (IV-1)

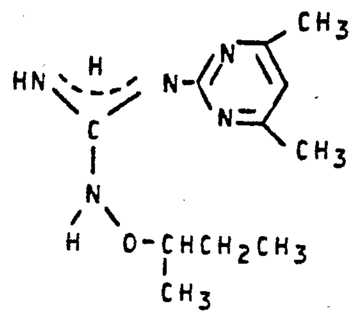

Eine Mischung aus 143 g (0,97 Mol) 2-Cyanamino-4,6-dimethyl-pyrimidin, 94,3 g (1,06 Mol) O-sec.-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57 % der Theorie) N'-(4,6-Dimethyl-pyrimidin-2-yl)-N''-sec.-butoxy-guanidin vom Schmelzpunkt 78°C.

Analog können die in der nachstehenden Tabelle 4 aufgeführten Verbindungen der Formel (IV) hergestellt werden:

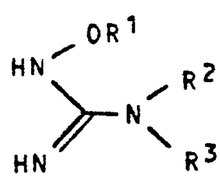

(IV)

Tabelle 4

| Beispiel Nr. | $R^1$ | $R^2$ | $R^3$ | Schmelzpunkt (°C) |
|---|---|---|---|---|
| (IV-2) | $-CH_2CH(CH_3)_2$ | H | 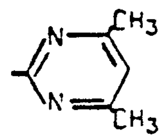 | 52 |
| (IV-3) | $-CH_2CH{=}CH_2$ | H | 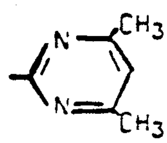 | 103 |
| (IV-4) | $-CH(CH_3)_2$ | H | 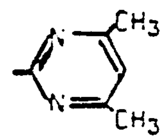 | |
| (IV-5) | 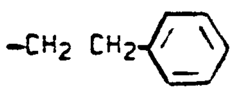 | H | 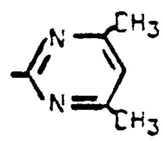 | $n_D^{24}$=1,5776 |
| (IV-6) | $-C_4H_9(n)$ | H | 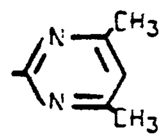 | $n_D^{20}$=1.5513 |
| (IV-7) | $-C_8H_{17}(n)$ | H | 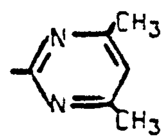 | 58 |
| (IV-8) | 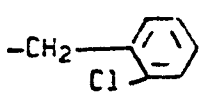 | H | 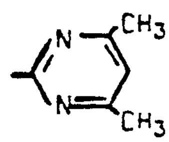 | 102-103 |
| (IV-9) | $-CH_2CH_2CH_2Cl$ | H | 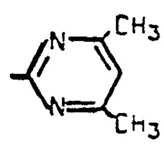 | 137 |
| (IV-10) | 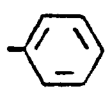 | H | 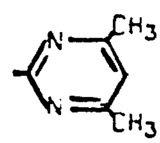 | 189-192 (Zers.) |

Tabelle 4 -Fortsetzung

| Beispiel Nr. | $R^1$ | $R^2$ | $R^3$ | Schmelzpunkt (°C) |
|---|---|---|---|---|
| (IV -11) | $-CH_2COOCH_3$ | H | 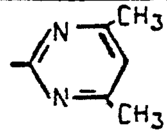 | 148-149 |
| (IV -12) | $-CH_2COOC_2H_5$ | H | 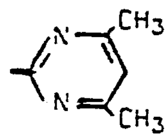 | 98- 99 |
| (IV -13) | 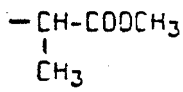 | H | 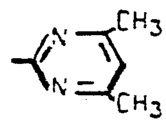 | 147-148 |
| (IV -14) | 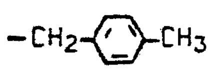 | H | 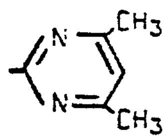 | 85- 86 |
| (IV -15) | 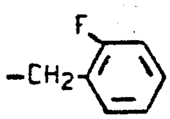 | H | 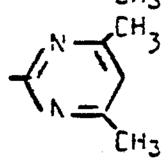 | 114-116 |
| (IV -16) | 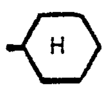 | H | 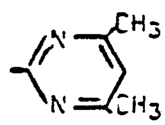 | |
| (IV -17) | 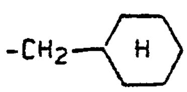 | H | 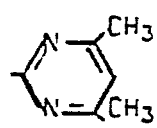 | |
| (IV -18) | $-CH_2CON(CH_3)_2$ | H | 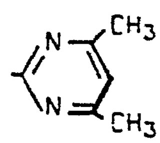 | |
| (IV -19) | $-CH_2OCH_3$ | H | 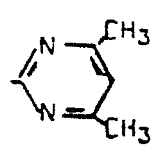 | |

Tabelle 4 - Fortsetzung

| Beispiel Nr. | $R^1$ | $R^2$ | $R^3$ | Schmelzpunkt (°C) |
|---|---|---|---|---|
| (IV-20) | $-CH_2SCH_3$ | H | 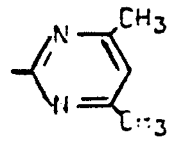 | |
| (IV-21) | 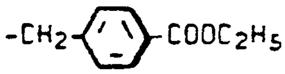 | H | 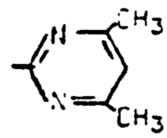 | 138 |
| (IV-22) | $-CH_2CF_3$ | H | 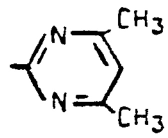 | |
| (IV-23) | 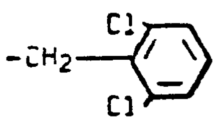 | H | 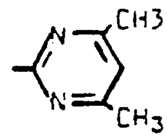 | 152 |
| (IV-24) | 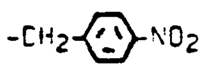 | H | 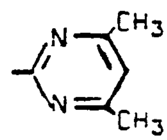 | 170-2 |
| (IV 25) | $-CH_3$ | H | 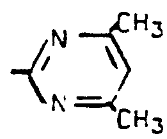 | 134-136 |
| (IV-26) | $-C_2H_5$ | H | 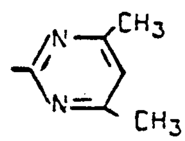 | 66 |
| (IV-27) | 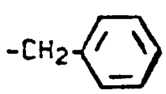 | H | 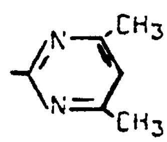 | 102 |
| (IV-28) | $-CH_3$ | $CH_3$ | 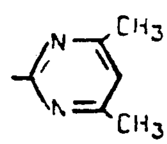 | 95 |

Tabelle 4- Fortsetzung

| Beispiel Nr. | $R^1$ | $R^2$ | $R^3$ | Schmelzpunkt (°C) |
|---|---|---|---|---|
| (IV-29) | $CH_3$ | $CH_3$ | 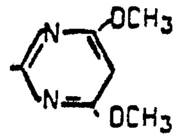 | 135 |
| (IV-30) | $CH_3$ | H | 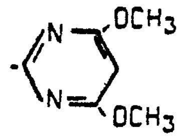 | 122 |
| (IV-31) | $CH_3$ | H | 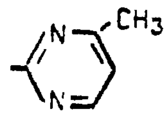 | 152 |
| (IV-32) | $CH_3$ | H | 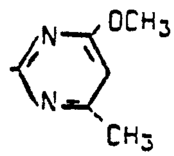 | 126 |
| (IV-33) | $CH_3$ | H | 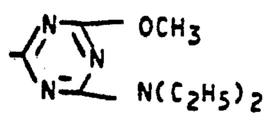 | 112 |
| (IV-34) | $CH_3$ | H | 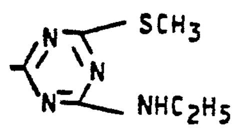 | 117 |
| (IV-35) | $C_2H_5$ | H | 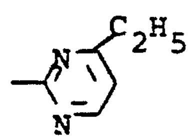 | |
| (IV-36) | $CH_3$ | H | 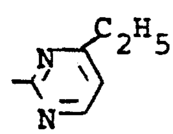 | 98 |
| (IV-37) | $-CH_2-$ 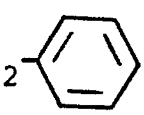 | H | 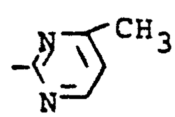 | 150 |

Tabelle 4 -Fortsetzung

| Beispiel Nr. | $R^1$ | $R^2$ | $R^3$ | Schmelzpunkt (°C) |
|---|---|---|---|---|
| (IV-38) | 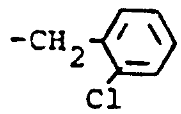 | H | 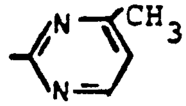 | 140 |
| (IV-39) | $CH_3$ | $CH_3$ | 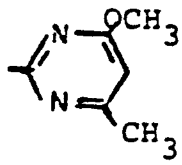 | 135 |
| (IV-40) | $CH_3$ | H | 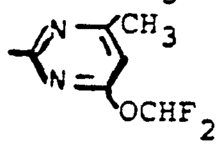 | |
| (IV-41) | $CH_3$ | H | 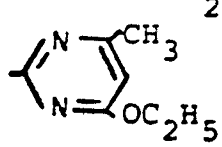 | |
| (IV-42) | $-\underset{CH_3}{CH}CH_2CH_3$ | H | 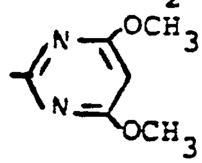 | 68 |
| (IV-43) | $-CH_2CH(CH_3)_2$ | H | 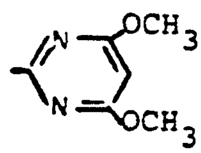 | 76 |
| (IV-44) | $-C_2H_5$ | H | 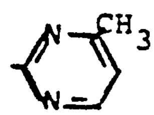 | 95 |
| (IV-45) | $-CH_2-COOC_2H_5$ | H | 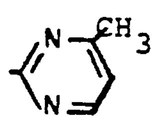 | |
| (IV-46) | $CH(C_6H_5)_2$ | H | 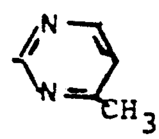 | 165 |

Tabelle 4 -Fortsetzung

| Beispiel Nr. | $R^1$ | $R^2$ | $R^3$ | Schmelz-punkt(°C) |
|---|---|---|---|---|
| (IV-47) | $CH_3$ | H | 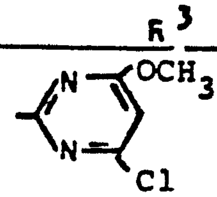 | 112 |
| (IV-48) | 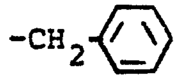 | H | 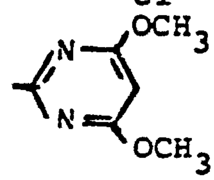 | 74 |
| (IV-49) | $-CH_3$ | H | 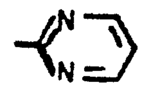 | 107-109 |
| (IV-50) | $-CH_3$ | H | 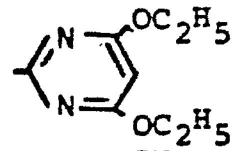 | |
| (IV-51) | 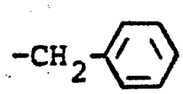 | H | 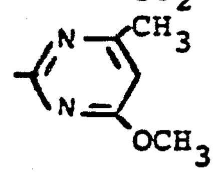 | $n_D^{20}$:1,5645 |
| (IV-52) | 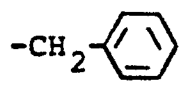 | H | 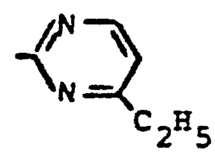 | 112 |
| (IV-53) | 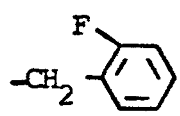 | H | 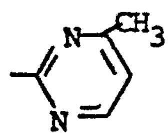 | 205 |
| (IV-54) | $-CH_2-CH_2-CH_2-Cl$ | H | 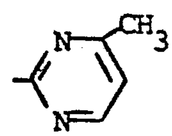 | ≈ 102 |
| (IV-55) | 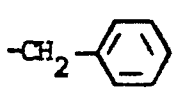 | H | 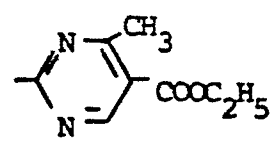 | 130 |
| (IV-56) | $-CH_2-CH_2-OCH_3$ | H | 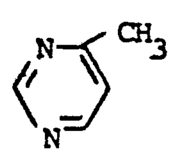 | 242 (Zers.) |

Tabelle 4 -Fortsetzung

| Beispiel Nr. | $R^1$ | $R^2$ | $R^3$ | Schmelzpunkt(°C) |
|---|---|---|---|---|
| (IV-57) | $-CH_2-CH=CH_2$ | H | 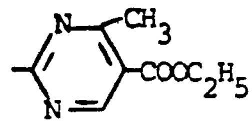 | 143 |
| (IV-58) | $-C_2H_5$ | H | 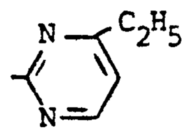 | 83 |
| (IV-59) | $-CH_3$ | H | 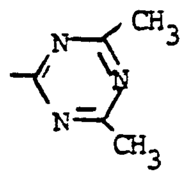 | 143 |
| (IV-60) | $-CH_3$ | H | 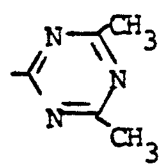 | 126 |
| (IV-61) | $-CH_3$ | H | 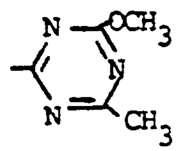 | 95 |
| (IV-62) | 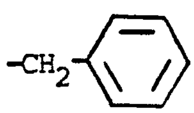 | H | 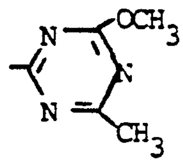 | 112 |
| (IV-63) | $-CH_2-CH_2-CH_2-CH_2-Cl$ | H | 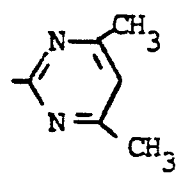 | amorph |
| (IV-64) | $-CH_2-CH_2-Cl$ | H | 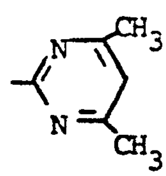 | amorph |
| (IV-65) | $-CH_2-COO\ CH(CH_3)_2$ | H | 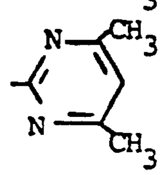 | 112 |

Tabelle 4 -Fortsetzung

| Beispiel Nr. | $R^1$ | $R^2$ | $R^3$ | Schmelzpunkt(°C) |
|---|---|---|---|---|
| (IV-66) | $-CH_2$ 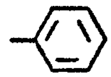 | H | 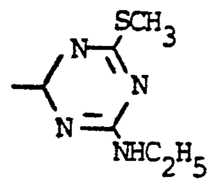 | 122 |
| (IV-67) | $-CH($ 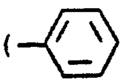 $)_2$ | H | 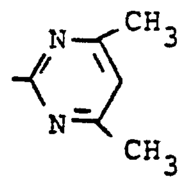 | 147-148 |

Herstellung der Ausgangsstoffe der Formel (V)

Beispiel V-1

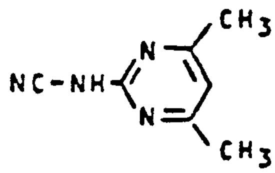

(V-1)

Ein Gemisch aus 42 g (0,5 Mol) Cyanoguanidin ('Dicyandiamid') und 50 g (0,5 Mol) 2,4-Pentandion ('Acetylaceton') wird 15 Stunden auf 120°C erhitzt. Dann wird nach Abkühlen das Reaktionsgemisch mit 500 ml Wasser versetzt und die Lösung bei 0°C bis 10°C mit Salzsäure angesäuert. Das hierbei kristallin angefallene Produkt wird durch Absaugen isoliert. Man erhält 51,8 g (70 % der Theorie) 2-Cyanamino-4,6-dimethyl-pyrimidin vom Schmelzpunkt 205°C.

Beispiel V-2

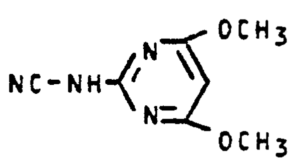

(V-2)

Eine auf 100°C erhitzte Lösung von 24 g (0,427 Mol) Kaliumhydroxid in 100 ml Wasser wird bei 100°C unter Rühren zu einer Mischung von 9,2 g (0,043 Mol) 4,6-Dimethoxypyrimidin-2-yl-thioharnstoff und 70 ml Wasser gegeben.

Man rührt 2 Minuten bei 100°C nach und gibt dann eine auf 100°C erwärmte Lösung von 16,2 g (0,05 Mol) Blei-II-acetat in 30 ml Wasser hinzu. Man erhitzt noch 5 Minuten unter Rückfluß, kühlt dann auf 0°C bis 5°C ab und versetzt die wäßrige Lösung mit 30 ml Eisessig. Das hierbei kristallin ausfallende Produkt wird durch Absaugen isoliert.

Man erhält 6,3 g (81,5 % der Theorie) 2-Cyanamino-4,6-dimethoxy-pyrimidin vom Schmelzpunkt 202°C.

Nach dem im vorausgehenden Beispiel exemplarisch beschriebenen Verfahren können die in der nachstehenden Tabelle 5 aufgeführten Verbindungen der Formel (V) hergestellt werden:

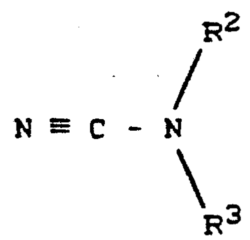

(V)

Tabelle 5:

| Bsp. Nr. | $R^2$ | $R^3$ | Schmelzpunkt (°C) |
|---|---|---|---|
| (V-3) | H | 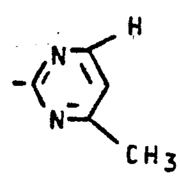 | 203 (Zer.) |

Tabelle 5 Fortsetzung

| Bsp. Nr. | $R^2$ | $R^3$ | Schmelz-punkt(°C) |
|---|---|---|---|
| (V-4) | H | 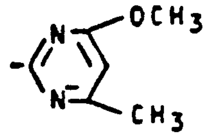 | 258 |
| (V-5) | H | 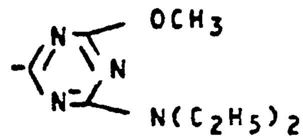 | 114 |
| (V-6) | H | 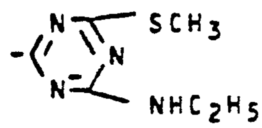 | |
| (V-7) | H | 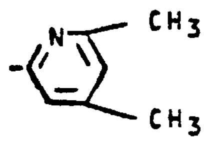 | 221(Zers.) |
| (V-8) | H | 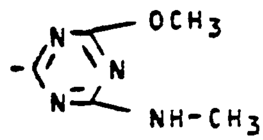 | 210 |
| (V-9) | H | 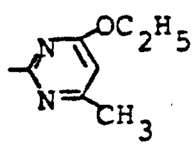 | |
| (V-10) | H | 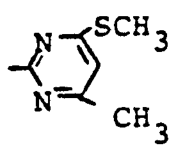 | |
| (V-11) | H | 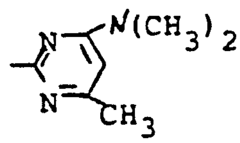 | |
| (V-12) | H | 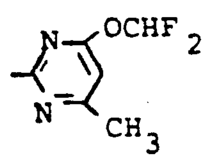 | 174 |

Tabelle 5 Fortsetzung

| Bsp. Nr. | $R^2$ | $R^3$ | Schmelz-punkt(°C) |
|---|---|---|---|
| (V-13) | H | 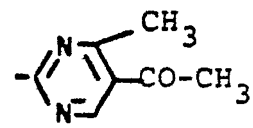 | 174 |
| (V-14) | H | 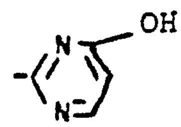 | >300 |
| (V-15) | H | 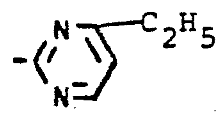 | 146 |
| (V-16) | H | 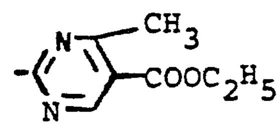 | 126 |
| (V-17) | H | 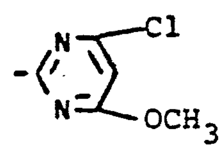 | 200 |
| (V-18) | H | 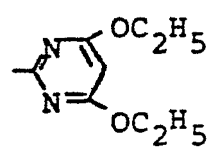 | 235-237 |
| (V-19) | H | 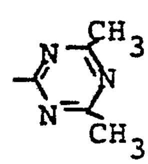 | 234 |
| (V-20) | H | 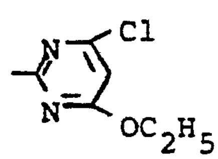 | |
| (V-21) | H | 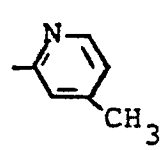 | 247-250 |

**Tabelle 5 Fortsetzung**

| Bsp. Nr. | $R^2$ | $R^3$ | Schmelzpunkt(°C) |
|---|---|---|---|
| (V-22) | H | 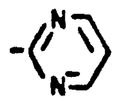 | 186 |
| (V-23) | H | 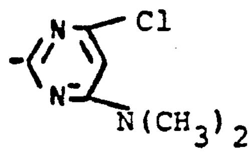 | |
| (V-24) | H | 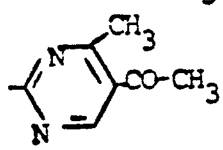 | 174 |
| (V-25) | 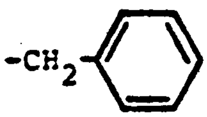 | 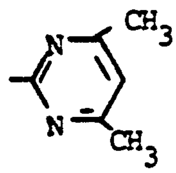 | 67-68 |
| (V-26) | H | 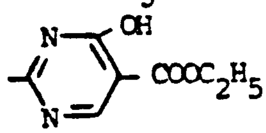 | 220 (Zers.) |
| (V-27) | H | 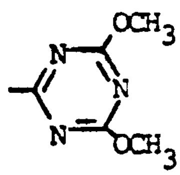 | > 300 |
| (V-28) | H | 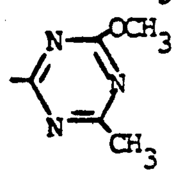 | 183 |
| (V-29) | H | 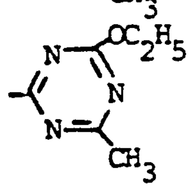 | > 200° (Zers.) |
| (V-30) | H | 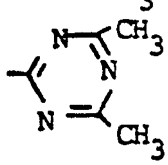 | 132 |

2-(Alkyl-cyano-amino)-pyrimidine der Formel (V) können beispielsweise wie folgt hergestellt werden:

Beispiel (V-31)

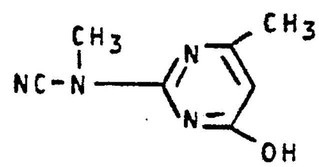

12,6 g (0,1 Mol) Dimethylsulfat werden zu einer Lösung von 15 g (0,1 Mol) 2-Cyanamino-4-hydroxy-6-methyl-pyrimidin und 4,1 g (0,1 Mol) Natriumhydroxid in 60 ml Wasser tropfenweise gegeben, wobei die Reaktionstemperatur von 20°C auf 40°C steigt. Nach zweistündigem Rühren bei 20°C wird das kristallin angefallene Produkt durch Absaugen isoliert.

Man erhält 11,1 g (68 % der Theorie) 2-(Methyl-cyan-amino)-4-hydroxy-6-methyl-pyrimidin vom Schmelzpunkt 290°C.

Analog erhält man:

Beispiel (V-32)

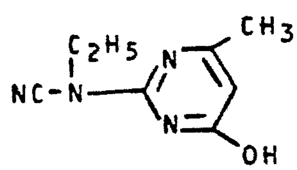

Fp: 215°C bis 220°C.

Beispiel (V-33)

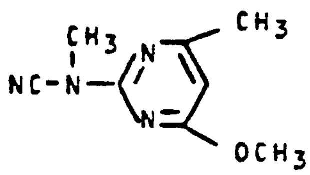

127,5 g (1 Mol) Dimethylsulfat werden zu einer Lösung von 75 g (0,5 Mol) 2-Cyanamino-4-hydroxy-6-methyl-pyrimidin - hergestellt nach Verfahren (b) - und 44 g (1,1 Mol) Natriumhydroxid in 750 ml Wasser tropfenweise gegeben, wobei die Reaktionstemperatur von 20°C auf 35°C ansteigt. Nach zwölfstündigem Rühren bei 20°C wird durch Zugabe von Natronlauge ein pH-Wert zwischen 9 und 10 eingestellt und das kristallin angefallene Produkt durch Absaugen isoliert.

Man erhält 13 g (15 % der Theorie) 2-(Methyl-cyano-amino)-4-methoxy-6-methyl-pyrimidin vom Schmelzpunkt 123°C.

Analog erhält man:

Beispiel (V-34)

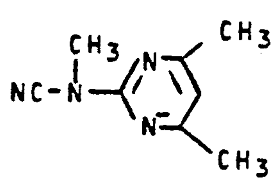

Fp: 104°C.

Beispiel (V-35)

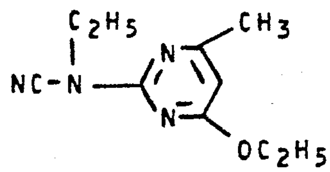

Fp: 71°C.

Herstellung der Ausgangsstoffe der Formel (XI)

Beispiel (XI-1)

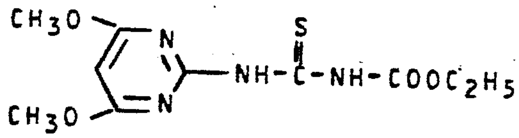

Eine Mischung aus 15,5 g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60°C gerührt. Dann wird auf 10°C abgekühlt, und das kristallin angefallene Produkt durch Absaugen isoliert.

Man erhält 22,5 g (79 % der Theorie) 1-(Ethoxycarbonyl)-3-(4,6-dimethoxy-pyrimidin-2-yl)-thioharnstoff vom Schmelzpunkt 194°C (Zers.).

Nach dem im vorausgehenden Beispiel exemplarisch beschriebenen Verfahren können die in der nachstehenden Tabelle 6 aufgeführten Verbindungen der Formel (XI) hergestellt werden:

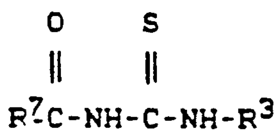

(XI)

Tabelle 6

| Beispiel Nr. | $R^7$ | $R^3$ | Schmelzpunkt(°C) |
|---|---|---|---|
| (XI -2) | 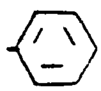 | 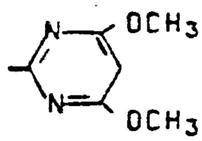 | 189 |
| (XI-3) | 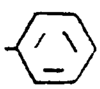 | 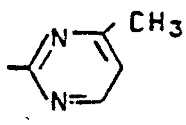 | 198-9 (Zers.) |
| (XI-4) | $-OC_2H_5$ | 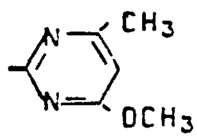 | 217 |
| (XI -5) | 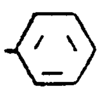 | 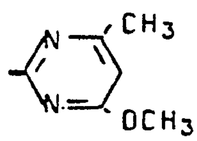 | 190 |
| (XI -6) | $-OC_2H_5$ | 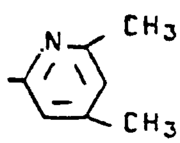 | 140 |
| (XI -7) | 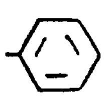 | 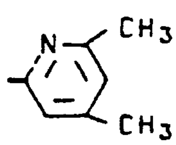 | 145 |

Tabelle 6 Fortsetzung

| Beispiel Nr. | $R^7$ | $R^3$ | Schmelzpunkt(°C) |
|---|---|---|---|
| (XI-8) | 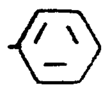 | 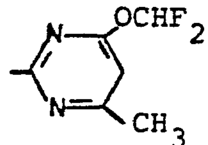 | 182 |
| (XI-9) | $-OC_2H_5$ | 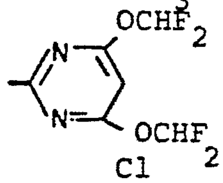 | 173 |
| (XI-10) | $-OC_2H_5$ | 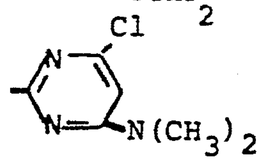 | 168 |
| (XI-11) | $-OC_2H_5$ | 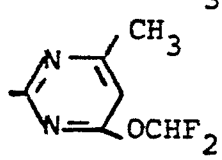 | 184-185 |
| (XI-12) | $-OC_2H_5$ | 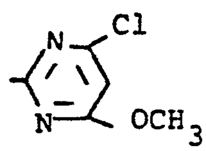 | 160-162 |
| (XI-13) | $-OC_2H_5$ | 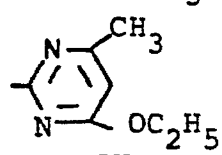 | |
| (XI-14) | $-OC_2H_5$ | 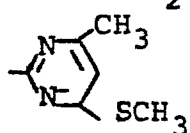 | |
| (XI-15) | $-OC_2H_5$ | 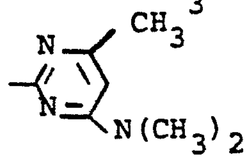 | |

Tabelle 6 Fortsetzung

| Beispiel Nr. | $R^7$ | $R^3$ | Schmelzpunkt (°C) |
|---|---|---|---|
| (XI-16) | 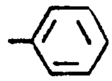 | 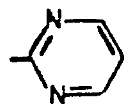 | 173 |
| (XI-17) | 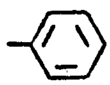 | 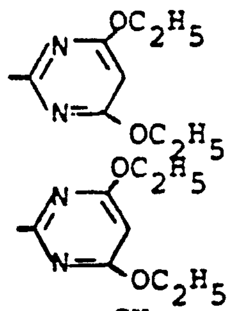 | 179 |
| (XI-18) | $-OC_2H_5$ |  | 159 |
| (XI-19) | 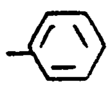 | 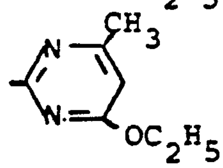 | 156 |
| (XI-20) | $-OC_2H_5$ | 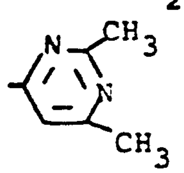 | 169 |
| (XI-21) | 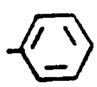 | 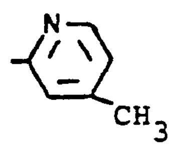 | 161 |
| (XI-22) | $-OC_2H_5$ | 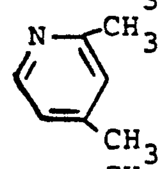 | 118-119 |
| (XI-23) | 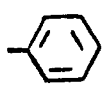 | 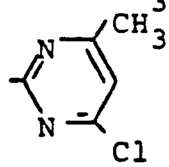 | 144 |
| (XI-24) | $-OC_2H_5$ | 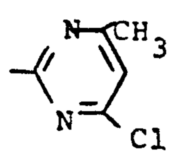 | 122-124 |

## Herstellung der Ausgangsstoffe der Formel (XII)

### Beispiel (XII-1)

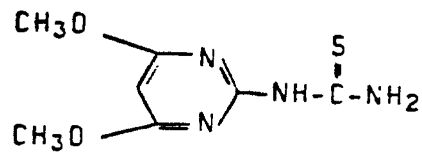

Eine Mischung aus 5,0 g (0,0175 Mol) 1-(Ethoxycarbonyl)-3-(4,6-dimethoxy-pyrimidin-2-yl)-harnstoff, 4,0 g (0,1 Mol) Natriumhydroxid und 100 ml Wasser wird 2 Tage bei 20°C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94 % der Theorie) 4,6-Dimethoxy-pyrimidin-2-yl-thioharnstoff vom Schmelzpunkt 245-8°C (Zers.).

Nach dem im vorausgehenden Beispiel exemplarisch beschriebenen Verfahren können die in der nachstehenden Tabelle 7 aufgeführten Verbindungen der Formel (XII) hergestellt werden:

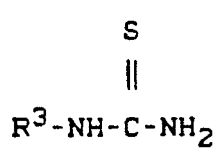

(XII)

Tabelle 7

| Beispiel Nr. | $R^3$ | Schmelzpunkt (°C) |
|---|---|---|
| (XII-2) | 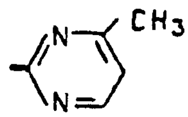 | 264-265 |
| (XII-3) | 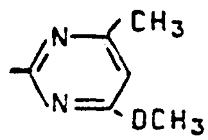 | 207 |
| (XII-4) | 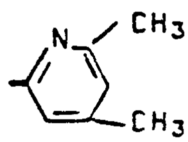 | 260 |
| (XII-5) | 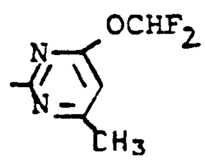 | 194 |
| (XII-6) | 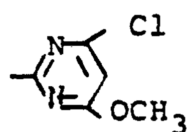 | 225-227 |
| (XII-7) | 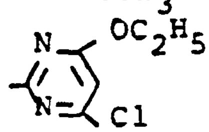 | |
| (XII-8) | 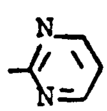 | 263 |
| (XII-9) | 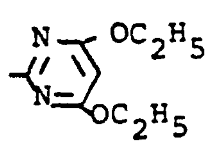 | 166 |
| (XII-10) | 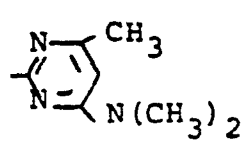 | |
| (XII-11) | 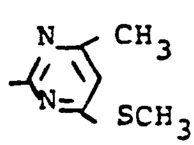 | |
| (XII-12) | 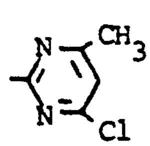 | > 180 (Zers.) |

## Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigten z.B. die folgenden Verbindungen der Herstellungsbeispiele (1) und (2) eine bessere herbizide Wirksamkeit als die aus dem Stand der Technik bekannte Verbindung (A).

## Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigten die folgenden Verbindungen der Herstellungsbeispiele (1) und (2) eine bessere herbizide Wirksamkeit als die aus dem Stand der Technik bekannten Verbindungen (A).

## Patentansprüche

1. 1-(2-Oxyaminocarbonylphenylsulfonyl)-3-heteroaryl-harnstoffe der allgemeinen Formel (I)

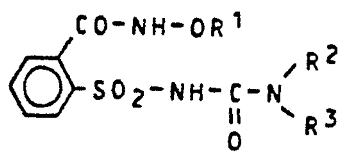

(I)

in welcher

$R^1$ für einen gegebenenfalls substituierten Rest aus der Reihe Alkyl, Alkenyl, Alkinyl, Cycloalkyl. Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für einen gegebenenfalls substituierten und/oder gegebenenfalls anellierten sechsgliedrigen aromatischen Heterocyclus, welcher wenigstens ein Stickstoffatom enthält, steht.

2. Verfahren zur Herstellung von 1-(2-Oxyaminocarbonylphenylsulfonyl-3-heteroaryl-harnstoffen der allgemeinen Formel (I) gemäß Anspruch 1, dadurch gekennzeichnet, daß man Heterocyclen der allgemeinen Formel (II)

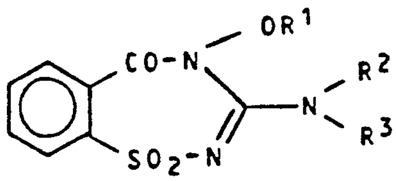

(II)

in welcher

$R^1$, $R^2$ und $R^3$ die oben angegebenen Bedeutungen haben,

mit Wasser in Gegenwart von Mineralsäuren und gegebenenfalls in Gegenwart von Verdünnungsmitteln bei Temperaturen zwischen 0°C und 100°C umsetzt.

3. Herbizide Mittel, gekennzeichnet durch einen Gehalt an mindestens einem 1-(2-Oxyaminocarbonylphenylsulfonyl)-3-heteroaryl-harnstoff der allgemeinen Formel (I) gemäß Anspruch 1.

4. Verwendung von 1-(2-Oxyaminocarbonylphenylsulfonyl)-3-heteroaryl-harnstoffen der allgemeinen Formel (I) gemäß Anspruch 1 zur Bekämpfung von unerwünschtem Pflanzenwachstum.

5. Verfahren zur Herstellung von herbiziden Mittel, dadurch gekennzeichnet, daß man 1-(2-Oxyaminocarbonylphenylsulfonyl)-3-heteroaryl-harnstoffe der allgemeinen Formel (I) gemäß Anspruch 1 mit Streckmitteln und/oder oberflächenaktiven Mitteln vermischt.